# EUROPEAN PATENT APPLICATION

(11) **EP 1 611 953 A1**
(43) Date of publication of application: **04.01.2006**
(21) Application number: 04725527.8
(22) Date of filing: 02.04.2004
(51) Int. Cl.: B01J 35/02, B01J 35/04, B01J 19/12, A61L 9/00, A61L 9/20, F24F 1/00

(54) **ULTRAVIOLET-RESPONSIVE THIN FILM PHOTOCATALYST AND APPLICATION THEREOF**

(30) Priority: 04.04.2003 JP 2003102343
(71) Applicant: Photo-catalytic Materials Inc., Komaki-shi, Aichi 485-0011 (JP)
(72) Inventor: Taoda, Hiroshi, c/o Chubu Center, Oaza-Shimoshidam (JP); Kato, Shigekazu, Komaki-shi, Aichi 485-0011 (JP)
(74) Representative: Ebner von Eschenbach, Jennifer
(86) International application number: PCT/JP2004/004874
(87) International publication number: WO 2004/089544

(57) **Abstract**

The present invention provides an ultraviolet-responsive thin film photocatalyst and an application thereof. The present invention relates to a transparent thin film titanium dioxide photocatalyst wherein the crystal size of the titanium dioxide catalyst forming the thin film is 5 nm to 50 nm, the adsorption wavelength peak is in the range of 200 nm to 300 nm and the film thickness is 0.1 to 1.0 microns, to the aforementioned photocatalyst wherein the crystal form of the titanium dioxide forming the thin film is a mixed state of spindle-shaped crystals and cubic crystals, to a filter wherein inorganic paper having silicon carbide (SiC) or amorphous silica (SiO₂) as a principal component or inorganic paper having activated charcoal, zeolite or sepiolite as a principal component is used as the substrate, and to an air sterile filtration device in which the aforementioned filter and a bactericidal ultraviolet lamp are combined.

## Description

### BACKGROUND OF THE INVENTION

### 1. Field of the Invention

The present invention relates to an ultraviolet-responsive thin film photocatalyst, and particularly relates to an ultraviolet-responsive thin film catalyst having a wavelength adsorption peak in the bactericidal ultraviolet range.

In addition to providing the aforementioned photocatalyst, the present invention is useful in that it provides a high-performance sterile filtration device which makes maximum use of both the bactericidal action of a bactericidal ultraviolet lamp using a bactericidal ultraviolet wavelength (253.7 nm) and the action of a photocatalyst having a wavelength adsorption peak in the same range.

### 2. Description of the Related Art

Because of the hydroxyl radicals, superoxide anions and other radicals generated when their surfaces are exposed to ultraviolet radiation, photocatalysts such as anatase titanium oxide and the like have been used as environmental purifiers for such functions as sterilization, antifouling, removal of aldehydes and other harmful substances and deodorizing and breaking down of malodorous substances specified by the Offensive Odor Control Law. Although many oxides can be used as photocatalysts, one common type of photocatalyst is titanium oxide, which has the three crystal forms anatase, rutile and brookite as well as an amorphous form, and of these anatase titanium dioxide is widely used because of its strong photocatalytic activity. The products which have been developed using this photocatalyst are too numerous to mention, but an example of one which uses the photocatalytic effect of titanium dioxide to prevent the growth of bacterial contaminants is a bacterial growth blocker in which a titania sol prepared from an alkoxide of titanium is coated on a substrate and baked to cover the substrate with a titanium oxide film (Japanese Patent No. 2883761, Specification).

However, the effective excitation range of anatase-type titanium dioxide photocatalysts starts at 388 nm, with an adsorption peak at a wavelength in the ultraviolet range of 350 to 365 nm, and an adsorption limit at 300 nm. By contrast, the most effective bactericidal wavelength for microorganisms such as bacteria and viruses is 253.7 nm. Consequently, it may not be possible to effectively excite the photocatalyst using a bactericidal ultraviolet lamp, while bactericidal ultraviolet cannot be used to control bacterial proliferation by means of a photocatalyst, so that in general the problem has been that photocatalysts can only act anti-bacterially, not bactericidally.

Under these circumstances and in light of the aforementioned related art, the inventors considered that maximum use could be made of the bactericidal action of a bactericidal ultraviolet lamp and the bactericidal effect of a photocatalyst if a photocatalyst could be prepared capable of using the bactericidal ultraviolet wavelength (253.7 nm) and having an effective excitation wavelength range in this range, and after exhaustive research they discovered that by forming a titanium dioxide photocatalyst as a thin film having a specific crystal structure a novel photocatalyst could be obtained having an effective excitation wavelength range in the aforementioned bactericidal ultraviolet range, and perfected the present invention as the result of further research.

### SUMMARY OF THE INVENTION

That is, it is an object of the present invention to provide a new type of photocatalyst which is excited by the aforementioned bactericidal ultraviolet wavelength (253.7 nm).

Moreover, it is also an object of the present invention to provide a new technology whereby by using a titanium dioxide photocatalyst in the form of a thin film having a specific crystal structure it is possible to excite the photocatalyst with the aforementioned bactericidal wavelength, so that the bactericidal effect of a bactericidal ultraviolet lamp and the photocatalytic effect of the titanium dioxide can be achieved in the same wavelength range.

Moreover, it is an object of the present invention to provide a thin film photocatalyst the wavelength adsorption peak of which is the bactericidal ultraviolet wavelength by forming an anatase titanium dioxide photocatalyst as a thin film having a specific crystal structure.

The present invention consists of the following technical means for solving these problems.
(1) A transparent thin film titanium dioxide photocatalyst, wherein the crystal size of the titanium dioxide catalyst forming the thin film is 5 nm to 50 nm, the adsorption wavelength peak is in the range of 200 nm to 300 nm and the film thickness is 0.1 to 1.0 microns.
(2) The photocatalyst according to (1) above, wherein the crystal form of the titanium dioxide forming the thin film is a mixed state of spindle-shaped crystals and cubic crystals.
(3) The photocatalyst according to (2) above, wherein the aforementioned crystals are dispersed in water or alcohol at a compounding ratio of 4:11.
(4) A filter, wherein the photocatalyst according to any of (1) through (3) above having an adsorption wavelength peak in the range of 200 nm to 300 nm is coated on the surface of a substrate.
(5) The filter according to (4) above, wherein inorganic paper having silicon carbide (SiC) or amorphous silica (SiO₂) as a principal component or inorganic paper having activated charcoal, zeolite or sepiolite as a principal component is used as the substrate.
(6) The filter according to (4) above, wherein a photocatalyst with an adsorption wavelength peak in the range of 200 nm to 300 nm is thin-film coated on the surface of a filter the substrate of which is molded in corrugated form.
(7) An air sterile filtration device, in which the filter according to (4) above and a bactericidal ultraviolet lamp are combined.
(8) The air sterile filtration device according to (7) above, wherein two or more filters are arranged parallel to the ultraviolet lamp at distances in the range of 5 mm to 15 mm.
(9) The air sterile filtration device according to (7) above, having an air migration path in which rather than being taken in directly perpendicular to the filter surface, air suctioned toward the filter flows first along the inner surface and then towards the outer surface of the filter or first along the outer surface and then towards the inner surface of the filter.

Next, the present invention is explained in more detail.

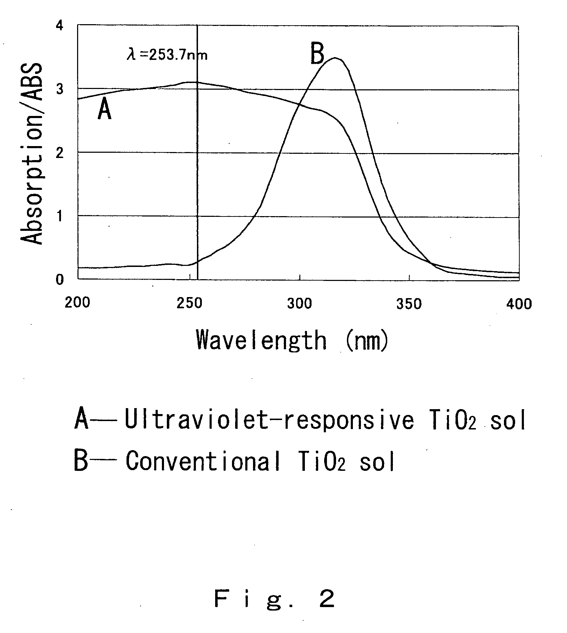

The ultraviolet-responsive titanium dioxide photocatalyst of the present invention has an ultraviolet adsorption peak near an ultraviolet wavelength of between 274 nm and 285 nm. The titanium dioxide used as the photocatalyst has a spindle-shaped crystal form (see Figure 2), and may be a mixture of spindle-shaped crystals and cubic crystals, and is preferably composed of crystals with a grain size of between 5 nm and 50 nm. The compounding ratio of spindle-shaped crystals to cubic crystals is preferably 4:11.

The photocatalyst of the present invention is preferably formed as a thin film, and is a transparent thin film with a thickness of 0.1 to 1.0 microns. A filter member consisting of inorganic paper having silicon carbide (SiC) or amorphous silica (SiO₂) as a principal component or inorganic paper having activated charcoal, zeolite or sepiolite as a principal component is preferably used as the substrate for forming the thin film, but the substrate is not limited thereto and another with the same effects could be used in the same way. Although not limited thereto, desirable examples of the form of the aforementioned substrate include a corrugated filter, a honeycomb filter and a ceramic filter composed of a three-dimensional silicon nitride framework.

In the present invention, the aforementioned filter may be combined with a bactericidal ultraviolet lamp to form an air sterile filtration device. In this case, two or more of the aforementioned filter members are preferably arranged parallel to the ultraviolet lamp at distances in the range of 5 mm to 15 mm, but they can be designed in any way according to the size, type and the like of the device. An air migration path is preferably provided wherein rather than being taken in directly perpendicular to the filter, air suctioned towards the filter passes along the inner surface of the filter towards the outer surface of the filter or along the outer surface of the filter towards the inner surface of the filter. The air sterile filtration device of the present invention comprises the aforementioned filter and ultraviolet lamp as essential constituent units, but other appropriate means which make up ordinary air sterile filtration devices may also be used without limits on their composition.

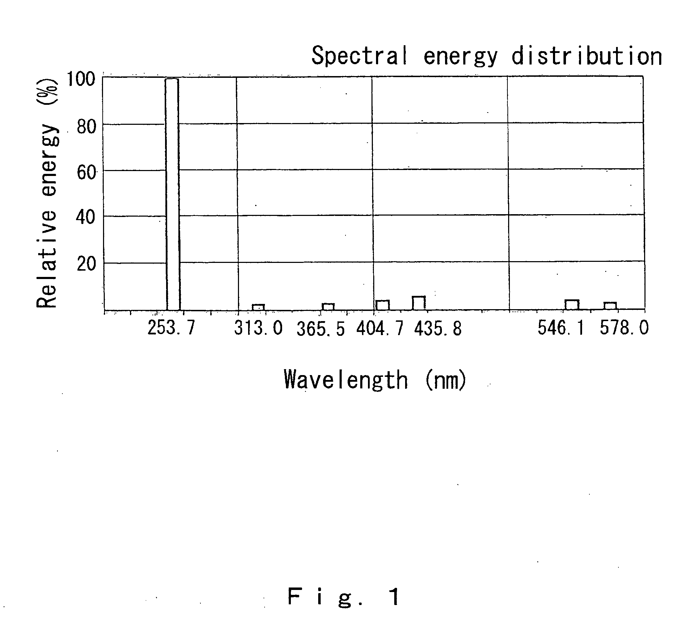

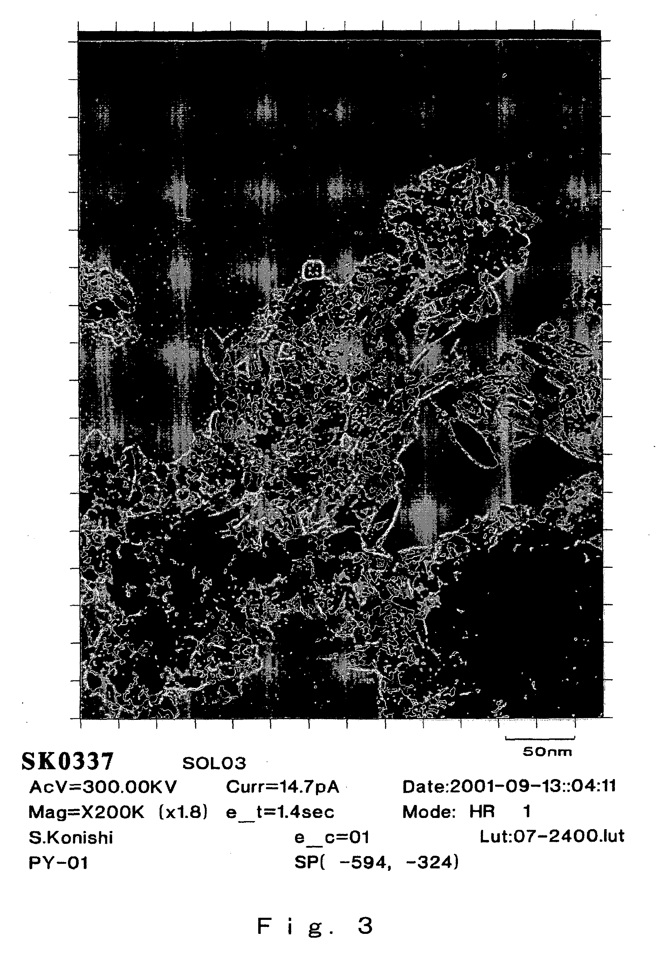

As shown in Figure 1, the spectral distribution of the bactericidal ultraviolet lamp has a peak at wavelength 253.7 nm. Moreover, as shown in Figure 2B, the effective excitation range of a conventional anatase titanium dioxide photocatalyst starts at 388 nm, with an adsorption peak at a wavelength in the ultraviolet range of 350 nm to 365 nm and an adsorption limited at 300 nm. By contrast, as shown in Figure 2A, the ultraviolet-responsive titanium dioxide photocatalyst of the present invention has an adsorption peak in the bactericidal ultraviolet range (253.7 nm). Moreover, as shown in Figure 3, the ultraviolet-responsive titanium dioxide catalyst of the invention of this application is composed of spindle-shaped crystals. Thus, the primary feature of the photocatalyst of the present invention, which is prepared with good reproducibility by a manufacturing method described in detail in the examples below, is that it has a completely different absorption curve from conventional titanium dioxide photocatalysts, with an absorption peak in the bactericidal ultraviolet range of 253.7 nm. The photocatalyst of the present invention is preferably useful for example as a germicidal, purifying and deodorizing filter element.

In the present invention, the thin film of photocatalyst is preferably formed by coating the sol prepared in the examples below to a specific thickness on a substrate, and baking it.

### BRIEF DESCRIPTION OF THE DRAWINGS

Figure 1 shows the spectral energy distribution of a bactericidal ultraviolet lamp.
Figure 2 shows the adsorption curve of an ultraviolet-responsive photocatalyst.
Figure 3 is a transmission electron microscope image of photocatalyst crystals.

### DESCRIPTION OF THE PREFERRED EMBODIMENTS

Next, the present invention is explained in detail based on examples, but the present invention is not in any way limited by the following examples.

### Example 1

### (1) Photocatalyst manufacture

750 ml of distilled water was placed in a preparation container (open 2L beaker), and vigorously agitated at about 400 rpm using a 140 φ. blade. 125 ml of TPT (Mitsubishi Gas Chemical) to which 20 ml of 2-propanol (Wako Pure Chemical) had been added was then dripped in at 5 ml/min. After completion of dripping, 7 ml of concentrated nitric acid (Wako Pure Chemical) was immediately added. This was hydrolyzed as is by agitation for 10 h at 80°C, with the 2-propanol being removed at the same time. The solution was initially cloudy white, but as hydrolysis progressed it became bluish-white and more transparent. The resulting contents had about 1/3 the initial volume. It was next autoclaved for 6 hours, at a temperature of 115°C or more. After completion, the contents had gelled, and this gel was agitated in a blender (capacity 1.2 L, maximum rotational speed 22,000 rpm). The TiO₂ content of the resulting sol was 14.7 wt%, and this was taken as sol A.

177.8 ml of 35% hydrogen peroxide (Mitsubishi Gas Chemical) was placed in a preparation container (open SUS container 200 φ x 270), and vigorous agitated at about 600 rpm using a 140 φ blade. 11.1 ml of TPT (Mitsubishi Gas Chemical) was then added all at once. A violent thermal reaction occurred, volatilizing the alcohol. After volatilization was complete, the rotational speed was reduced to about 200 rpm, and 782.5 ml of distilled water was added followed by 122.3 ml of 35% hydrogen peroxide. After 0.5 hours' agitation, 31.5 ml of a 1N sodium hydroxide solution was added. About 1,100 ml of a yellow viscous liquid or gel was obtained. This was autoclaved for 6 hours at a temperature of 110°C or more. The contents upon completion were about 920 ml or about 75% of the initial volume. The Ti content of this sol was 1.96 wt%, and the TiO₂ content was 3.28 wt%. Sol A was dripped into the resulting sol at 15 ml/min to a pH of 6.5 to 7.5. The volume ratio was approximately 93:7. The result was left sealed for 12 hours. It was initially a light yellow liquid, but produced a white precipitate, and at this point the reaction was considered complete. The TiO₂ content was 4.26 wt%. The adsorption curve of the resulting photocatalyst is shown in Figure 1, while a transmission electron microscope image of the photocatalyst crystals is shown in Figure 2.

### Example 2

A device was prepared using a filter in combination with a bactericidal ultraviolet light to evaluate bactericidal effect.

Immediately upon completion of a 2.1 x 10⁷ CFU/ml x 10 ml spray test using *Mycobacterium bovis* (BCG Tokyo) as the bacterium, the paper HEPA filters used in bacterial collection and detection were immersed in Middlebrook 7H9 liquid medium, and ultrasound treated for 1 minute. Next, a 10x dilution sequence was prepared using the same Middlebrook 7H9 liquid medium in each case, and 0.1 ml/plate of each of the dilutions was seeded on two plates of Middlebrook 7H10 agar flat plate medium. Each liquid medium and agar flat-plate medium was cultured continuously for 4 weeks at 37°C. The results are shown in Table 1.

In the two tests of test section 4 in which bacterial culture was positive, the bacterial count from the Paper HEPA Filter (3.4 to 3.7 x 10³ CFU/ml) was 1/1000 or less of the initial spray volume (average 2.1 x 10⁷ CFU/ml). This suggests that fairly efficient bacterial and dust collection effects can be achieved simply with the honeycomb element of the "Pleasant" air cleaner.

**Table 1**

| | Test conditions | | Filter used | Spray time | Culture results | Notes |
|---|---|---|---|---|---|---|
| 1 | Photocatalyst + ultraviolet | High blower | No.327 | 14 min. | (-), (-) | |
| 1' | Photocatalyst + ultraviolet | Medium blower | No.327 | 14 min. | (-), (-) | |
| 2 | Photocatalyst + ultraviolet | Medium blower | No.2 | 14 min. | (-), (-) | |
| | Photocatalyst + ultraviolet | Medium blower | No.2 | 14 min. | (-), (-) | |
| 3 | Photocatalyst + ultraviolet | High blower | No.2 | 14 min. | (-), (-) | |
| | Photocatalyst + ultraviolet | High blower | No.2 | 14 min. | (-), (-) | |
| 4 | Photocatalyst + no light source | High blower | No.2 | 14 min. | (+), (+) | With intermission |
| | Photocatalyst + no light source | High blower | No.2 | 14 min. | (+), (+) | |

### Example 2

Decontamination tests were performed to examine bactericidal capacity using a device prepared using a filter in combination with a bactericidal ultraviolet light. The results are shown in Table 2.

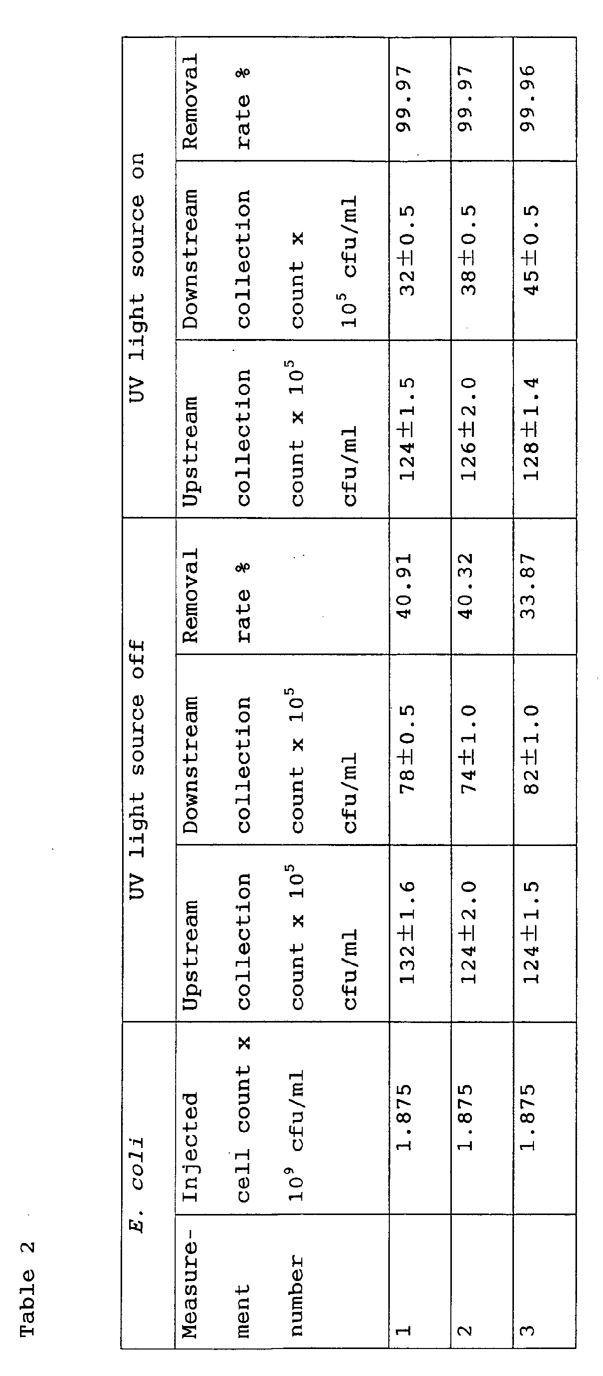

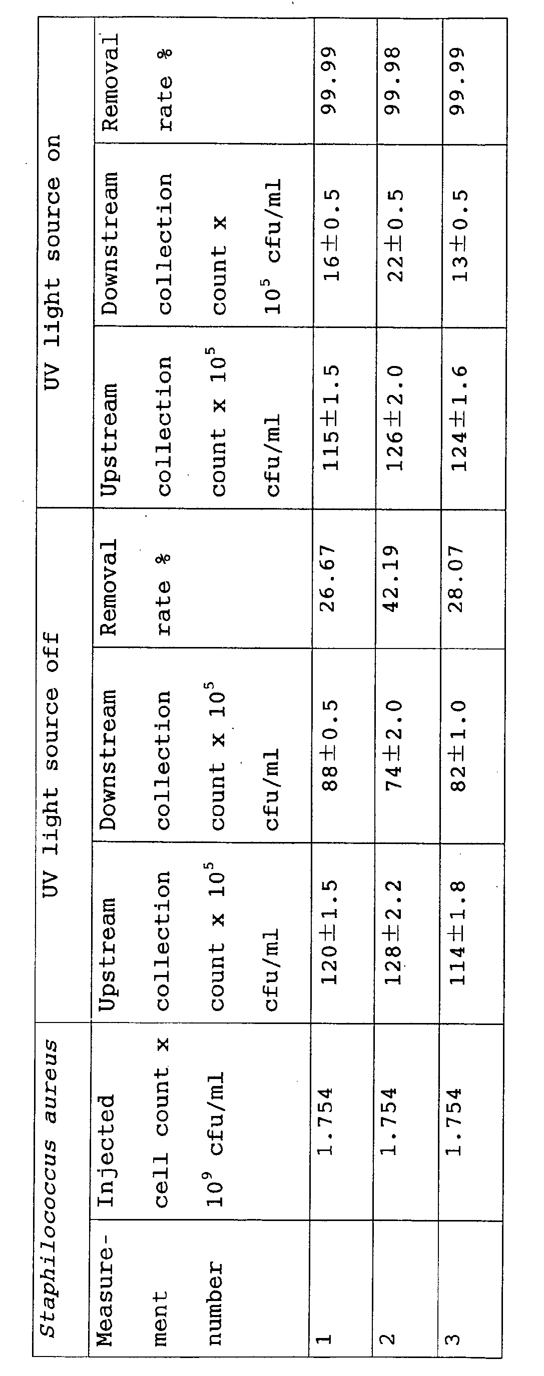

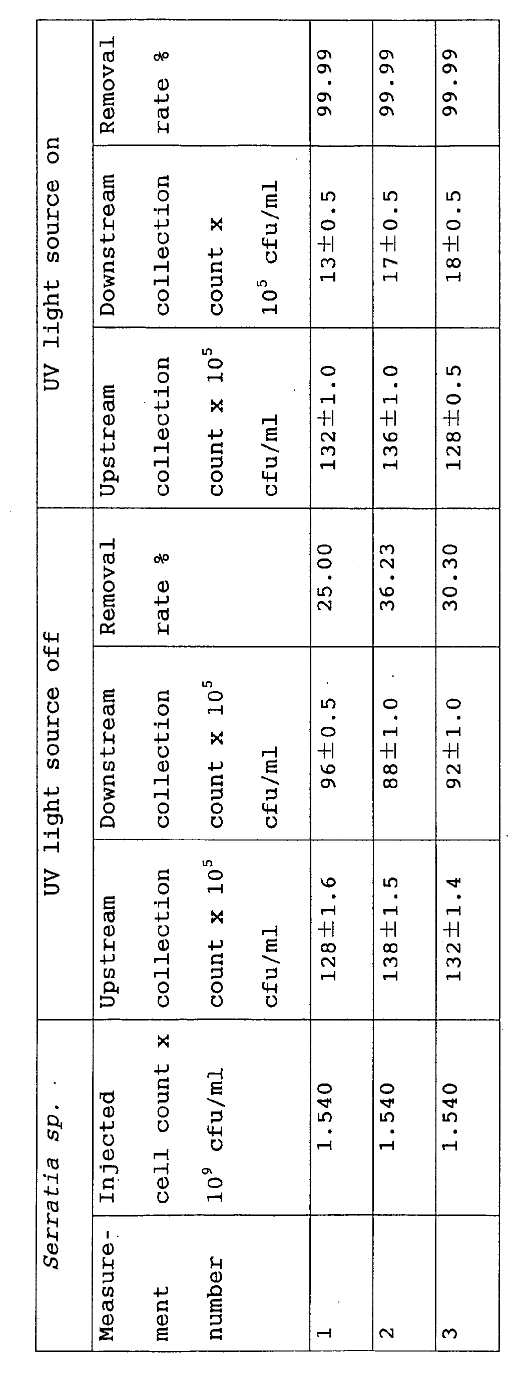

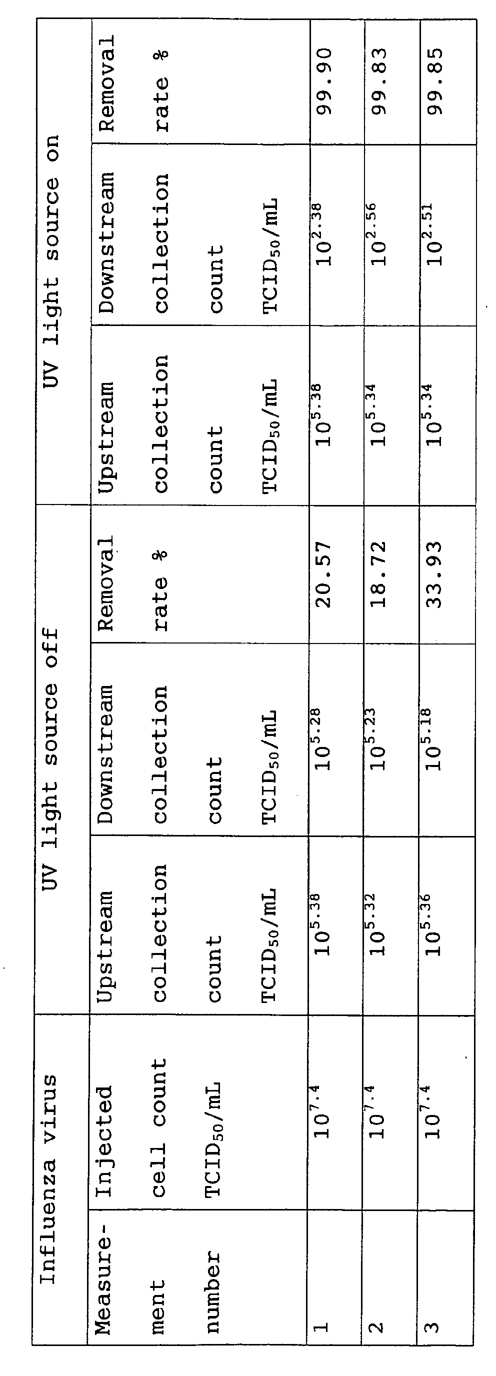

As described in detail above, the present invention relates to an ultraviolet-responsive photocatalyst and an application thereof, and the following effects can be achieved with the present invention: (1) a photocatalyst the wavelength absorption peak of which is a bactericidal ultraviolet wavelength can be manufactured by forming a titanium dioxide catalyst as a thin film with a specific crystal form, (2) a photocatalyst the wavelength absorption peak of which is a bactericidal ultraviolet wavelength can be provided, (3) by combining a bactericidal ultraviolet lamp with the aforementioned ultraviolet-responsive photocatalyst, a novel bactericidal method and device can be provided which make maximum use of the photocatalytic effect and the bactericidal effect of the bactericidal ultraviolet lamp, and (4) an air sterile filtration device can be provided using this photocatalyst.

## Claims

1. A transparent thin film titanium dioxide photocatalyst, wherein the crystal size of the titanium dioxide catalyst forming the thin film is 5 nm to 50 nm, the adsorption wavelength peak is in the range of 200 nm to 300 nm and the film thickness is 0.1 to 1.0 microns.

2. The photocatalyst according to Claim 1, wherein the crystal form of the titanium dioxide forming the thin film is a mixed state of spindle-shaped crystals and cubic crystals.

3. The photocatalyst according to Claim 2, wherein said crystals are dispersed in water or alcohol at a compounding ratio of 4:11.

4. A filter, wherein the photocatalyst according to any of Claims 1 through 3 having an adsorption wavelength peak in the range of 200 nm to 300 nm is coated on the surface of a substrate.

5. The filter according to Claim 4, wherein inorganic paper having silicon carbide (SiC) or amorphous silica (SiO₂) as a principal component or inorganic paper having activated charcoal, zeolite or sepiolite as a principal component is used as the substrate.

6. The filter according to Claim 4, wherein a photocatalyst with an adsorption wavelength peak in the range of 200 nm to 300 nm is thin-film coated on the surface of a filter the substrate of which is molded in corrugated form.

7. An air sterile filtration device, in which the filter according to Claim 4 and a bactericidal ultraviolet lamp are combined.

8. The air sterile filtration device according to Claim 7, wherein two or more filters are arranged parallel to the ultraviolet lamp at distances in the range of 5 mm to 15 mm.

9. The air sterile filtration device according to Claim 7, having an air migration path in which rather than being taken in directly perpendicular to the filter surface, air suctioned toward the filter flows first along the inner surface and then towards the outer surface of the filter or first along the outer surface and then towards the inner surface of the filter.
